# EUROPEAN PATENT SPECIFICATION

(11) **EP 4 333 700 B1**
(45) Date of publication and mention of the grant of the patent: **17.09.2025**
(21) Application number: 22724766.5
(22) Date of filing: 24.04.2022
(51) Int. Cl.: A61B 5/055, A61B 5/00, A61B 5/0205, A61B 5/11, A61B 6/04, A61G 7/00, A61B 5/16, A61B 6/03, A61N 5/10

(54) **MEDICAL IMAGING SYSTEM**
SYSTEM ZUR MEDIZINISCHEN BILDGEBUNG
SYSTÈME D'IMAGERIE MÉDICALE

(30) Priority: 04.05.2021 EP 21172081
(43) Date of publication of application: 13.03.2024
(73) Proprietor: Koninklijke Philips Electronics N.V., 5656 AE Eindhoven (NL)
(72) Inventor: LEUSSLER, Christoph Günther, 5656 AG Eindhoven (NL); NORDHOFF, Tanja, 5656 AG Eindhoven (NL)
(74) Representative: Philips Intellectual Property & Standards
(86) International application number: PCT/EP2022/060791
(87) International publication number: WO 2022/233611

(56) References cited:
- EP-A1- 3 649 924
- US-A1- 2014 357 981
- US-A1- 2016 089 054

## Description

### FIELD OF THE INVENTION

The present invention relates to a medical imaging system, a medical imaging method, as well as to a computer program element.

### BACKGROUND OF THE INVENTION

US2016/089054A1 describes a magnetic resonance system, useful for imaging a patient, comprising: (a) a magnetic resonance device for imaging a patient, comprising an open bore, the magnetic resonance device at least partially contained in an envelope comprising in its circumference at least one recess; and, (b) an MRI-safe cart made of MRI-safe material, comprising a substantially horizontal base and at least one substantially horizontal incubator above the base, the base and the incubator are interconnected by at least one pillar. At least a portion of the cart and the magnetic resonance device are configured to fit together such that at least a portion of the incubator is reversibly housed within the magnetic resonance device, and further at least a portion of the base is reversibly housed within at least one recess.

US2014/357981A1 describes a patient table adapted for use in association with an MR scanner for neonatal infants. The patient table has an extendable patient bed attached to and extendable from the patient table. The patient bed may be at least partially inserted into an MR scanner without requiring the patient table to enter the MR scanner. A transport mechanism is on the underside of the patient table so that it may be readily moved over the floor on which it rests. The patient table includes a latching mechanism that may operate to releasably attach the patient table to a patient table docking assembly. The docking assembly is operative to selectively move the patient table towards, into and away from the MR scanner.

High patient throughput is crucial for many medical imaging facilities. At the same time, patient engagement and experience are becoming more important and are even part of the reimbursement in selected markets like the US. Moreover, imaging will become more and more autonomous in future with less operator depended actions and automated workflow steps. This leads to the need of good supporting tools to inform and guide operator and patient scenarios.

FlexTrak Mammo and the wide bore Ingenia MR system provide a comprehensive breast solution. Easy patient preparation, workflow and after-care is combined with high image quality, patient comfort and scanner efficiency. FlexTrak Mammo is available in combination with the dS Breast 16ch coil and/or dS Breast 7ch coil. An adjustable head rest with patient mirror and soft patient ramp are included to promote patient comfort.

Upcoming RF wireless coil technology applied in MRI signal transmission simplifies coil handling by the patient and operator for autonomous image acquisition. While wireless RF coil technology offers convenience and ease of operation, it introduces challenges in maintaining the phase information of individual signals, or powering the active parts of the RF coil. The combination of wireless coils, and detection of optimal relative overlap is a further challenge to be addressed. For autonomous preparation of a patient, different combinations of receive coil setups need to be selected as a function of region to be imaged or individually selected anatomy.

There is a need to resolve these issues.

### SUMMARY OF THE INVENTION

It would be advantageous to have an improved means of improving the throughput and patient comfort with respect to medical imaging. The object of the present invention is solved with the subject matter of the independent claims, wherein further embodiments are incorporated in the dependent claims. It should be noted that the following described aspects and examples of the invention apply to the medical imaging system, and also to the medical imaging method as well as to the computer program element.

In a first aspect, there is provided a medical imaging system, comprising:
- a movable bed;
- a medical imaging scanner;
- at least one sensor; and
- a processor unit;

wherein the movable bed is configured to move a patient on the movable bed from a first area of a medical centre to a second area of the medical centre where the medical imaging scanner is located;
wherein the medical imaging scanner is intended to acquire at least one medical image of the patient;wherein the at least one sensor is configured to acquire sensor information relating to the patient on the movable bed, and
wherein the at least one sensor is configured to acquire the sensor information relating to the patient on the movable bed prior to movement of the patient from the first area of the medical centre to the second area of the medical centre and/or during movement of the patient from the first area of the medical centre to the second area of the medical centre;
wherein the at least one sensor is configured to provide the sensor information to the processor unit;
wherein the medical imaging scanner is configured to provide scanner status information for the medical imaging scanner to the processor unit;wherein the processor unit is configured to determine control information for the movable bed, the determination comprising utilization of the sensor information and the scanner status information; and
wherein the processor unit is configured to utilize the control information to provide a communication that an operator is required to come to the movable bed and /or the processor unit is configured to utilize the control information to provide a communication that an operator is required to move the movable bed and/or the processor unit is configured to utilize the control information to provide a communication that an operator is required to stop moving the movable bed.

In other words prior to moving the patient from the first area of the medical centre to the second area of the medical centre and/or during movement of the patient from the first area of the medical centre to the second area of the medical centre, at least one sensor can acquire sensor information relating to the patient on the movable bed. Also, prior to moving the patient from the first area of the medical centre to the second area of the medical centre and/or during movement of the patient from the first area of the medical centre to the second area of the medical centre, scanner status information for the medical imaging scanner can be provided to the processor unit. Additionally, prior to moving the patient from the first area of the medical centre to the second area of the medical centre and/or during movement of the patient from the first area of the medical centre to the second area of the medical centre, the processing can determine control information for the movable bed, the determination comprising utilization of the sensor information and the scanner status information.

Thus, patient workflow with respect to timely utilization of a medical image scanner is improved, because information relating to both the patient on a bed to be used to transport the patient to the scanner, derived from sensor data, and status information relating to the scanner is utilized, thereby enabling the patient to be delivered to the scanner at exactly the correct time for the scan.

In this manner, if the scanner is expected to be free for use earlier than expected, the patient can be expeditiously brought to the scanner, and conversely if a scan for a patient undergoing a scan is taking longer than necessary, the patient can be kept away from the scanner room until the scanner is ready.

Furthermore, by monitoring the patient, if the patient is not ready for a scan, for example sensor data shows that they have become agitated or stressed, they can be held back from being scanned, enabling another patient to "jump the queue" ahead of the agitated stressed patient, ensuring that the scanner's time is utilized to its maximum. Also, when the patient is determined to now be ready for the scan, they can re-join the queue and even jump to the front of the queue to minimise the amount of time they will have to wait for the scan.

It is to be noted that the second area of the medical centre can be for example comprises a mobile medical image scanner, such as a mobile MRI, installed on a truck. Thus, a patient can be moved from within a building to a truck. This also means that "medical centre" is here utilized in a general sense in terms of meaning an area where a patient is moved from one area or location where a medical image scanner is not located to an area or location where the medical image scanner is located.

In an example, the sensor information relating to the patient on the movable bed comprises vital signs data for the patient on the movable bed.

In an example, the vital signs data comprises one or more of: ECG data; anaesthesia data; heart beat data; blood pressure data; skin conductance data; breathing rate data; motion data of body parts determined from RF and/or optical sensing.

Thus, the physiological and/or emotional state of the patient can be taken into account.

In an example, the medical imaging scanner is an MRI image unit, an X-ray image unit, a PET image unit, a CT-Radiation therapy unit, or a MR-LINAC scanner.

In an example, the medical imaging scanner is an MRI image unit, and the at least one sensor comprises at least one wireless MRI compatible coil. The sensor information relating to the patient on the movable bed can then comprise status information relating to the at least one wireless MRI compatible coil.

In an example, the status information relating to the at least one wireless MRI compatible coil comprises one or more of: battery status of the wireless MRI compatible coil or coils; type of the coil or coils; the coil vendor or vendors; RF power limit of the coil or coils; operational temperature of the coil or coils; service data of the coil or coils; functionality data of the coil or coils.

In this manner, wireless MRI coils that are starting to be used with MRI scans can be set up beforehand, and positioned with respect to the patient during transit of the patient to the scanner and not delay scanning by undertaking this set-up at the scanner.

In an example, the control information for the movable bed comprises coil configuration information to configure the wireless MRI compatible coil with respect to the acquisition of the at least one medical image of the patient.

In an example, the processor unit is configured to determine configuration information for the medical imaging scanner. The determination comprises utilization of the sensor information and the scanner status information.

In an example, the scanner status information for the medical imaging scanner comprises an indication of whether the scanner is in use and/or an indication when the scanner will be available for use.

In this manner, the control information can take into account when the scanner is expected to be ready for the patient, enabling information/instructions/control to be provided to enable the bed with the patient to be brought to the scanner at exactly the correct time for the scan, and be kept out of the way until that time.

In an example, the system comprises one or more further movable beds. The scanner status information can then comprise sensor information relating to one or more other patients on the one or more further movable beds, and the medical imaging scanner is intended to acquire at least one medical image for each of the one or more other patients.

Thus, the system can take account of all the patients in their various stages of transit to the scanner, changing the patient scan order if necessary to allow agitated/stressed patients to calm down and prioritise cases such as emergencies or children, and plan the scans based on the readiness of equipment to be used in the scan etc.

In an example, the control information comprises instructions to control one or more brakes for one of more wheels of the movable bed.

In an example, the control information comprises instructions to control the provision of movement information to an operator. The movement information comprises information displayed on at least one visual display unit.

In an example, the control information comprises instructions to control the provision of movement information to an operator. The movement information comprises information output on at least acoustic output unit.

In an example, the at least one visual display unit comprises a visual display unit mounted to the movable bed.

In an example, the at least one visual display unit comprises a visual display unit separated from the movable bed.

In an example, the at least one acoustic output unit comprises an acoustic output unit mounted to the movable bed.

In an example, the at least one acoustic output unit comprises an acoustic output unit separated from the movable bed.

In a second aspect, there is provided a medical imaging method with a medical imaging system, the method comprising:
a) moving a patient on a movable bed from a first area of a medical centre to a second area of the medical centre where a medical imaging scanner is located, and wherein the medical imaging scanner is intended to acquire at least one medical image of the patient;
b) prior to moving the patient and/or during movement of the patient acquiring by at least one sensor sensor information relating to the patient on the movable bed;
c) providing the sensor information to a processor unit;
d) prior to moving the patient and/or during movement of the patient providing scanner status information for the medical imaging scanner to the processor unit; and
e) prior to moving the patient and/or during movement of the patient determining by the processor unit control information for the movable bed, the determining comprising utilizing the sensor information and the scanner status information, and utilizing by the processing unit the control information to provide a communication that an operator is required to come to the movable bed and /or utilizing by the processing unit the control information to provide a communication that an operator is required to move the movable bed and/or utilizing by the processing unit the control information to provide a communication that an operator is required to stop moving the movable bed.

According to another aspect, there is provided a computer program element controlling one or more of the systems as previously described which, if the computer program element is executed by a processing unit, is adapted to perform the method as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of a medical imaging system;
Fig. 2 shows a medical imaging method;
Fig. 3 shows an example of a patient bed with wireless access and integrated sensing;
Fig. 4 shows an example of patient preparation using a wireless link patient tray, where individual patient data (motion, breathing) is checked via wireless link;
Fig. 5 shows an example of communication of individual mobile patient bed with a state machine (or processor unit) via wireless link, where the individual data is used to control the MRI sequence, inform remote or local operator actions (MRI console), and connect to 3D sensing; and
Fig. 6 shows an example of a detailed workflow or algorithm of wireless link connection and control of workflow via feedback software loop.

### DETAILED DESCRIPTION OF EMBODIMENTS

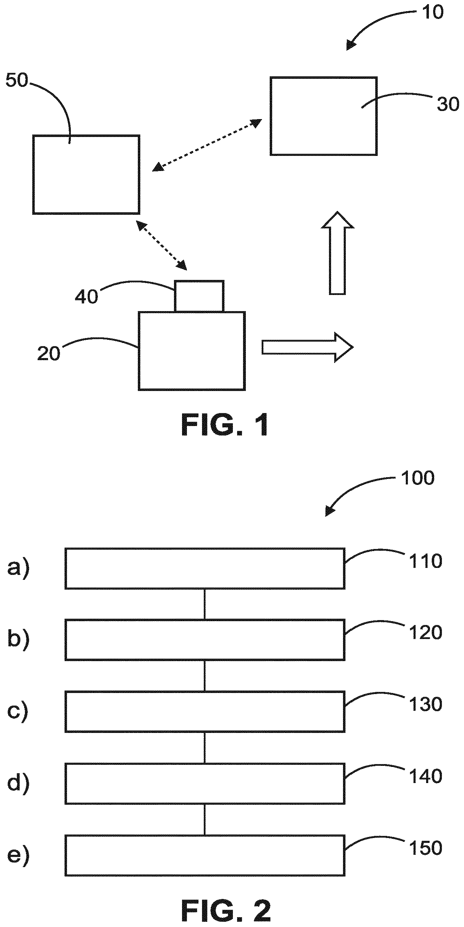

Fig. 1 shows a schematic example of a medical imaging system 10. The system comprises a movable bed 20, a medical imaging scanner 30, at least one sensor 40, and a processor unit 50. The movable bed is configured to move a patient on the movable bed from a first area of a medical centre to a second area of the medical centre where the medical imaging scanner is located. The medical imaging scanner is intended to acquire at least one medical image of the patient. The at least one sensor is configured to acquire sensor information relating to the patient on the movable bed. The at least one sensor is configured to provide the sensor information to the processor unit. The medical imaging scanner is configured to provide scanner status information for the medical imaging scanner to the processor unit. The processor unit is configured to determine control information for the movable bed, the determination comprising utilization of the sensor information and the scanner status information.

In an example, the processor unit is configured to provide the sensor information to the medical imaging scanner.

In an example, the processor unit is configured to provide the control information to the medical imaging scanner.

Thus, the medical imaging scanner, in terms of a controller or processing unit for the scanner, is also provided with a view of what is happening with respect to patients and their transit to the scanner.

The processor unit is configured to utilize the control information to provide a communication that an operator is required to come to the movable bed.

The processor unit is configured to utilize the control information to provide a communication that an operator is required to move the movable bed.

The processor unit is configured to utilize the control information to provide a communication that an operator is required to stop moving the movable bed.

In an example, the processor unit is contained with the movable bed.

In an example, the processor unit is separate to the movable bed.

In an example, the processor unit is configured to wirelessly communicate with the at least one sensor.

In an example, the processor unit is configured to wirelessly communicate with the medical imaging scanner.

In an example, the at least one sensor is connected to the movable bed.

In an example, the at least one sensor is separate to the movable bed.

In an example, the at least one sensor is configured to wirelessly communicate with the processor unit.

In an example, the at least one sensor is configured to wirelessly communicate with the medical imaging scanner.

For example, communication to the medical imaging scanner can be via intermediate repeater transponders distributed in the imaging centre. The medical imaging scanner can be connected via a digital network, cloud state machine with the transponders.

According to an example, the sensor information relating to the patient on the movable bed comprises vital signs data for the patient on the movable bed.

According to an example, the vital signs data comprises one or more of: ECG data; anaesthesia data; heart beat data; blood pressure data; skin conductance data; breathing rate data; motion data of body parts determined from RF and/or optical sensing.

In an example, the processor unit is configured to utilize the vital signs data to provide a communication that a medical expert and/or operator is required to come to the movable bed.

According to an example, the medical imaging scanner is an MRI image unit, an X-ray image unit, a PET image unit, a CT-Radiation therapy unit, or a MR-LINAC scanner.

According to an example, the medical imaging scanner is an MRI image unit, and the at least one sensor can comprise at least one wireless MRI compatible coil. The sensor information relating to the patient on the movable bed can then comprise status information relating to the at least one wireless MRI compatible coil.

According to an example, the status information relating to the at least one wireless MRI compatible coil comprises one or more of: battery status of the wireless MRI compatible coil or coils; type of the coil or coils; the coil vendor or vendors; RF power limit of the coil or coils; operational temperature of the coil or coils; service data of the coil or coils; functionality data of the coil or coils.

In an example, the at least one wireless MRI compatible coil comprises a plurality of wireless MRI compatible coils, and the status information comprises the number of coils on the patient.

In an example, the at least one wireless MRI compatible coil comprises a plurality of wireless MRI compatible coils, and the status information comprises their position on the patient.

In an example, the at least one wireless MRI compatible coil comprises a plurality of wireless MRI compatible coils, and the status information comprises their position relative to one another.

In an example, the at least one wireless MRI compatible coil comprises a plurality of wireless MRI compatible coils, and the status information comprises the number of coils on the patient, and their position on the patient and their position relative to one another.

Thus, individual coil elements can be deactivated if it is determined that the coil positioning is not optimal.

According to an example, the control information for the movable bed comprises coil configuration information to configure the wireless MRI compatible coil with respect to the acquisition of the at least one medical image of the patient.

In an example, the coil configuration information comprises one or more of: at least one switching time; digital filter characteristics; analogue filter characteristics; RF noise matching adaptation, integrated sensor activation characteristics; bandwidth; field strength; gradient strength, B0 static field, RF coil loop activation.

According to an example, the processor unit is configured to determine configuration information for the medical imaging scanner, the determination comprising utilization of the sensor information and the scanner status information.

In an example, the configuration information for the medical imaging scanner comprises a MRI scan sequence for the patient.

According to an example, the scanner status information for the medical imaging scanner comprises an indication of whether the scanner is in use and/or an indication when the scanner will be available for use.

According to an example, the system comprises one or more further movable beds, and the scanner status information comprises sensor information relating to one or more other patients on the one or more further movable beds, and the medical imaging scanner is intended to acquire at least one medical image for each of the one or more other patients.

According to an example, the control information comprises instructions to control one or more brakes for one of more wheels of the movable bed.

According to an example, the control information comprises instructions to control the provision of movement information to an operator, and the movement information comprises information displayed on at least one visual display unit. Additionally or alternatively the control information comprises instructions to control the provision of movement information to an operator, and the movement information comprises information output on at least acoustic output unit.

According to an example, the at least one visual display unit comprises a visual display unit mounted to the movable bed. Additionally or alternatively the at least one visual display unit comprises a visual display unit separated from the movable bed;

According to an example, the at least one acoustic output unit comprises an acoustic output unit mounted to the movable bed. Additionally or alternatively the at least one acoustic output unit comprises an acoustic output unit separated from the movable bed.

In an example, the control information controlling the brakes of the bed can be correlated with the control information providing visual and/or acoustic information to an operator, thus to inform about workflow status.

In an example, the medical imaging scanner is intended to acquire the at least one medical image of the patient whilst the patient is lying on at least a part of the movable bed.

In other words, the movable bed can dock with the medical imaging scanner and the patient can be transferred from the movable bed to a bed of the scanner, however the movable bed, or at least a part of the movable bed, is compatible with the medical imaging scanner enabling the patient to remain on the bed, or at least a part of the bed, whilst being imaged.

In an example, a first part of the movable bed on which the patient lies is configured to detach from a second part of the movable bed, and wherein the first part of the movable bed is configured to enable the at least one medical image of the patient to be acquired whilst the patient is lying on the first part of the movable bed.

Thus, a display with a display mounted on the bed can indicate to an operator that the bed should now be moved, and this can be displayed visually to the operator and this can be accompanied by an acoustic signal or even synthesized voice to also inform the operator to move the bed, in case they are not looking at the display. At the same time, brakes on a wheel or wheels of the bed can be released enabling the bed to be moved. At an appropriate point the operator is informed to stop moving the bed, and the brakes applied to stop the bed from moving. The brakes can be applied in a gradual manner, rather than off/on enabling the operator to be informed due to the gradually increasing resistance that the bed should now be placed in a stationary orientation. Additionally or alternatively to the display and loudspeaker on the bed, the operator can have a portable unit, similar to a smartphone that informs them to move the bed and how and to where it should be moved.

Fig. 2 shows a medical imaging method 100 with a medical imaging system 10. The method is shown in its basic steps, and where step a) or at least parts of step a) can occur before or at the same time as a number of different steps as described below, and the method comprises:
in a moving step 110, also referred to as step a) moving a patient on a movable bed from a first area of a medical centre to a second area of the medical centre where a medical imaging scanner is located, and wherein the medical imaging scanner is intended to acquire at least one medical image of the patient;
   prior to moving the patient and/or during movement of the patient in an acquiring step 120, also referred to as step b), acquiring, by at least one sensor, sensor information relating to the patient on the movable bed;
in a providing step 130, also referred to as step c), providing the sensor information to a processor unit;
prior to moving the patient and/or during movement of the patient in a providing step 140, also referred to as step d), providing scanner status information for the medical imaging scanner to the processor unit; and
prior to moving the patient and/or during movement of the patient in a determining step 150, also referred to as step e), determining by the processor unit control information for the movable bed, the determining comprising utilizing the sensor information and the scanner status information, information, and utilizing by the processing unit the control information to provide a communication that an operator is required to come to the movable bed and /or utilizing by the processing unit the control information to provide a communication that an operator is required to move the movable bed and/or utilizing by the processing unit the control information to provide a communication that an operator is required to stop moving the movable bed.

In an example, the sensor information relating to the patient on the movable bed comprises vital signs data for the patient on the movable bed.

In an example, the vital signs data comprises one or more of: ECG data; anaesthesia data; heart beat data; blood pressure data; skin conductance data; breathing rate data; motion data of body parts determined from RF and/or optical sensing.

In an example, the medical imaging scanner is an MRI image unit, an X-ray image unit, a PET image unit, a CT-Radiation therapy unit, or a MR-LINAC scanner.

In an example, the medical imaging scanner is an MRI image unit, and the at least one sensor can comprises a wireless MRI compatible coil. The sensor information relating to the patient on the movable bed can comprise status information relating to the wireless MRI compatible coil.

In an example, the status information relating to the wireless MRI compatible coil comprises one or more of: battery status of the wireless MRI compatible coil; type of coil; coil vendor; RF power limit of the coil; operational temperature of the coil; service data of the coil; functionality data of the coil.

In an example, the control information for the movable bed comprises coil configuration information to configure the wireless MRI compatible coil with respect to the acquisition of the at least one medical image of the patient.

In an example, the coil configuration information comprises one or more of: at least one switching time; digital filter characteristics; analogue filter characteristics; RF noise matching adaptation, integrated sensor activation characteristics; bandwidth; field strength; gradient strength, B0 static field, RF coil loop activation.

In an example, prior to moving the patient and/or during movement of the patient the method comprises determining by the processor unit configuration information for the medical imaging scanner, the determining comprising utilizing the sensor information and the scanner status information.

In an example, the configuration information for the medical imaging scanner comprises a MRI scan sequence for the patient.

In an example, the scanner status information for the medical imaging scanner comprises an indication of whether the scanner is in use and/or an indication when the scanner will be available for use.

In an example, the scanner status information comprises sensor information relating to one or more other patients on one or more further movable beds, and wherein the medical imaging scanner is intended to acquire at least one medical image for each of the one or more other patients.

In an example, the control information comprises instructions to control one or more brakes for one of more wheels of the movable bed.

In an example, the control information comprises instructions to control the provision of movement information to an operator, wherein the movement information comprises information displayed on at least one visual display unit.

In an example, the at least one visual display unit comprises a visual display unit mounted to the movable bed; and/or wherein the at least one visual display unit comprises a visual display unit separated from the movable bed.

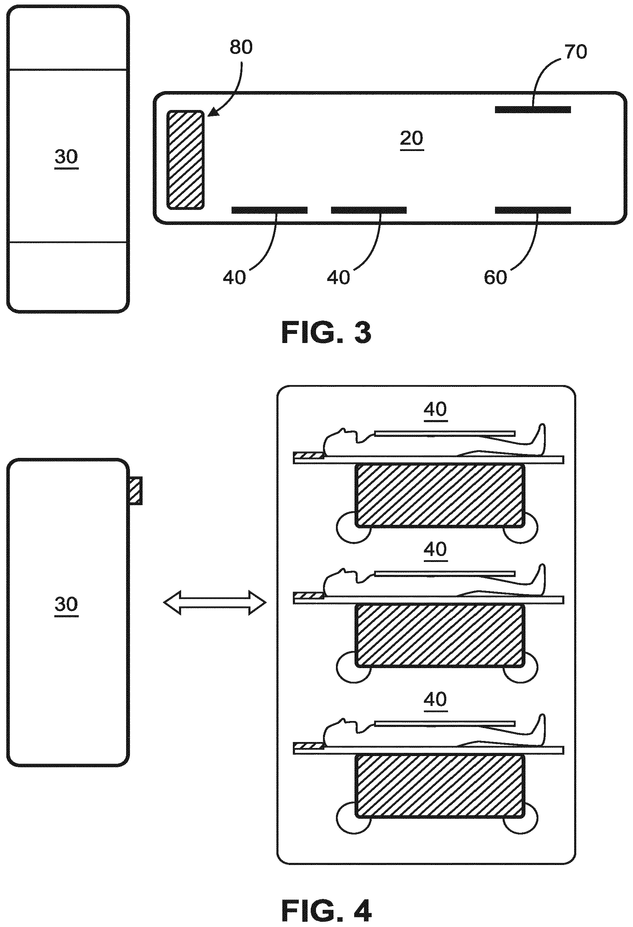

The medical imaging system and medical imaging method are explained in specific detail, where reference is made to Figs. 3-6. The specific embodiments centre on MRI imaging, but a system with other imaging modalities can also apply.

The inventors were aware of the following problems that existed with respect to the workflow aspects of coordinating patients for medical imaging in an efficient and timely manner, whilst maximizing the patient's comfort and experience.

Patient bed does have electrical connectors
RF coil check after coils are connected
B1 system power as a function of coil
When patient is prepared for example for mammo trak, no coil connection or sensing
Functionality of coils is not checked
Several/Multiple patients cannot be prepared together
System needs optical/galvanic connection (connectors)
Wireless coils can be used on the patient and sensors can also be utilized, but functionality checks and placement indications have to be done when the patient is at the scanner causing a delay
No independent power source and no wireless link to remote operator
For multiple patients, each patient needs to be prepared and checked separately Monitoring of prepared patients
The new patient transfer bed and associated functionality within what can be termed a new overall medical imaging system was developed by the inventors to overcome these problems.

In summary, these problems were overcome through a new movable patient support or bed, which has an autonomous integrated wireless link allowing patient preparation and controlled workflow. Multiple patients can be prepared together, or at least a new patient can start to be prepared before an earlier patient has arrived at the scanner. RF coils and bed integrated sensors can checked remotely for vital signs data and to check if patient is ready for a scan. The dataflow is routed via a wireless link to a remote host (state machine or processor unit). To control the patient bed workflow, individual patient beds are equipped with electronically controlled brakes and display information to only allow the correct sequence of patients arriving at the scanner. Results of the individual measured data is fed to a state machine (processor unit), which configures the MR sequence and external control and projection elements. Communication of individual mobile patient bed with the state machine is via wireless link. The individual data is used to control, load individual MRI sequences, inform remote or local operator (MRI console) and connect/communicate to 3D sensing. An algorithm of wireless link connection and control of workflow is via feedback software loop.

Thus, wireless coils and sensing along with scanner status information enables the use of a mechanical patient bed/tray/support, which needs no connectors or cable routing. The patient bed has a wireless interface integrated and DC power battery, and information on the scanner and the patient is utilized to coordinate a patient's arrival at the scanner with respect to other patients. This allows for multiple guided patient preparation and a faster workflow

As shown in Figs. 3-4, a patient bed with a wireless link and integrated sensing is shown. The mobile patient bed is moved to the scanner and docking is directly performed without any galvanic or optical connection allowing faster and safe workflow. The MRI scanner is configured regarding individual coil and patient data. Multiple patients can be prepared simultaneously. Each patient has individual coils equipment, mattress and sensing. The individual state and workflow is automatically documented and checked with a patient data base.

In Fig. 3:
20 indicates patient bed wireless access,
30 indicates MRI scanner,
40 indicates sensor,
60 indicates switch,
70 indicates coil connect, and
80 indicates wireless link transceiver / battery.

In Fig. 4:
30 indicates MRI scanner,
40 indicates wireless coil, and
The imagery on the right represents the patients being prepared for scanning away from the scanner.

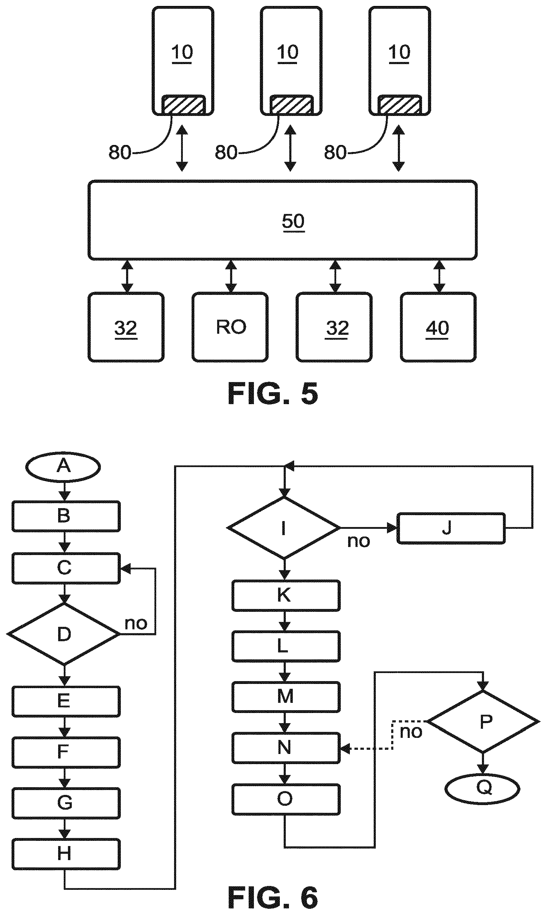

As shown in Figs. 5-6, the state machine (processor unit) directly processes the vital signs data and presents the information as textual or symbolic information. The data is continuously updated for the operator and MRI scanner. The operator obtains feedback information about motion or preceding events or user interactions and visualized one or more attribute parameter thresholds linked with a vital signs camera. The vital signs camera can be a 3D optical camera, 3D LIDAR or a 3D RF RADAR sensor. Detection of thresholds also come from the at least one sensor on the patient bed. If for example an anaesthesia unit is not ready, or going in service mode, then an appropriate action can to be taken by the computer program on the processing unit.

An algorithm of wireless link connection and control of workflow via feedback software loop runs on the state machine (processor unit).

In Fig. 5:
10 indicates bed 1, bed 2, and bed 3,
30 indicates MRI scanner,
32 indicates MRI console,
40 indicates 3D sensing,
50 indicates wireless link state machine (processor unit), and
RO indicates a remote operator.

In Fig, 6:
A indicates start
B indicates wireless link connect
C indicates prepare patient
D indicates ready
E indicates check coils
F indicates check vital signs
G indicates wireless link transmission
H indicates adapt and scan protocol
I indicates MRI ready
J indicates wait
K indicates transport
L indicates docking
M indicates load sequence
N indicates start scan
O indicates run protocols
P indicates MRI scan ready
Q indicates stop

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment, not claimed as such, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While the invention has been illustrated and described in detail in the drawings and foregoing description, such illustration and description are to be considered illustrative or exemplary and not restrictive. The invention is not limited to the disclosed but it is defined by the appended claims.

## Claims

1. A medical imaging system (10), comprising:
- a movable bed (20);
- a medical imaging scanner (30);
- at least one sensor (40); and
- a processor unit (50);
wherein the movable bed is configured to move a patient on the movable bed from a first area of a medical centre to a second area of the medical centre where the medical imaging scanner is located;
wherein the medical imaging scanner is intended to acquire at least one medical image of the patient;
wherein the at least one sensor is configured to acquire sensor information relating to the patient on the movable bed, and wherein the at least one sensor is configured to acquire the sensor information relating to the patient on the movable bed prior to movement of the patient from the first area of the medical centre to the second area of the medical centre and/or during movement of the patient from the first area of the medical centre to the second area of the medical centre;
wherein the at least one sensor is configured to provide the sensor information to the processor unit;
wherein the medical imaging scanner is configured to provide scanner status information for the medical imaging scanner to the processor unit;
**characterized in that**
the processor unit is configured to determine control information for the movable bed, the determination comprising utilization of the sensor information and the scanner status information; and
**in that** the processor unit is configured to utilize the control information to provide a communication that an operator is required to come to the movable bed and /or the processor unit is configured to utilize the control information to provide a communication that an operator is required to move the movable bed and/or the processor unit is configured to utilize the control information to provide a communication that an operator is required to stop moving the movable bed.

2. System according to claim 1, wherein the sensor information relating to the patient on the movable bed comprises vital signs data for the patient on the movable bed.

3. System according to claim 2, wherein the vital signs data comprises one or more of: ECG data; anaesthesia data; heart beat data; blood pressure data; skin conductance data; breathing rate data; motion data of body parts determined from RF and/or optical sensing.

4. System according to any of claims 1-3, wherein the medical imaging scanner is an MRI image unit, an X-ray image unit, a PET image unit, a CT-Radiation therapy unit, or a MR-LINAC scanner.

5. System according to claim 4, wherein the medical imaging scanner is an MRI image unit, and wherein the at least one sensor comprises at least one wireless MRI compatible coil, and wherein the sensor information relating to the patient on the movable bed comprises status information relating to the at least one wireless MRI compatible coil.

6. System according to claim 5, wherein the status information relating to the at least one wireless MRI compatible coil comprises one or more of: battery status of the wireless MRI compatible coil or coils; type of the coil or coils; the coil vendor or vendors; RF power limit of the coil or coils; operational temperature of the coil or coils; service data of the coil or coils; functionality data of the coil or coils.

7. System according to any of claims 5-6, wherein the control information for the movable bed comprises coil configuration information to configure the wireless MRI compatible coil with respect to the acquisition of the at least one medical image of the patient.

8. System according to any of claims 1-7, wherein the processor unit is configured to determine configuration information for the medical imaging scanner, the determination comprising utilization of the sensor information and the scanner status information.

9. System according to any of claims 1-8, wherein the scanner status information for the medical imaging scanner comprises an indication of whether the scanner is in use and/or an indication when the scanner will be available for use.

10. System according to any of claims 1-9, wherein the system comprises one or more further movable beds, and wherein the scanner status information comprises sensor information relating to one or more other patients on the one or more further movable beds, and wherein the medical imaging scanner is intended to acquire at least one medical image for each of the one or more other patients.

11. System according to any of claims 1-10, wherein the control information comprises instructions to control one or more brakes for one of more wheels of the movable bed.

12. System according to any of claims 1-11, wherein the control information comprises instructions to control the provision of movement information to an operator, wherein the movement information comprises information displayed on at least one visual display unit; and/or wherein the movement information comprises information output on at least acoustic output unit.

13. System according to claim 12, wherein the at least one visual display unit comprises a visual display unit mounted to the movable bed; and/or wherein the at least one visual display unit comprises a visual display unit separated from the movable bed; and/or wherein the at least one acoustic output unit comprises an acoustic output unit mounted to the movable bed; and/or wherein the at least one acoustic output unit comprises an acoustic output unit separated from the movable bed.

14. A medical imaging method (100) with a medical imaging system (10), the method comprising:
a) moving (110) a patient on a movable bed from a first area of a medical centre to a second area of the medical centre where a medical imaging scanner is located, and wherein the medical imaging scanner is intended to acquire at least one medical image of the patient;
b) prior to moving the patient and/or during movement of the patient acquiring (120) by at least one sensor sensor information relating to the patient on the movable bed;
c) providing (130) the sensor information to a processor unit;
d) prior to moving the patient and/or during movement of the patient providing (140) scanner status information for the medical imaging scanner to the processor unit; and
e) prior to moving the patient and/or during movement of the patient determining (150) by the processor unit control information for the movable bed, the determining comprising utilizing the sensor information and the scanner status information, and utilizing by the processing unit the control information to provide a communication that an operator is required to come to the movable bed and /or utilizing by the processing unit the control information to provide a communication that an operator is required to move the movable bed and/or utilizing by the processing unit the control information to provide a communication that an operator is required to stop moving the movable bed.

15. A computer program element for controlling a system according to any one of claims 1 to 13, which when executed by a processor is configured to carry out the method of claim 14.

## Patentansprüche

1. Medizinisches Bildgebungssystem (10), umfassend:
- ein bewegliches Bett (20);
- einen medizinischen Bildgebungsscanner (30);
- zumindest einen Sensor (40); und
eine Prozessoreinheit (50);
wobei das bewegliche Bett dazu konfiguriert ist, einen Patienten auf dem beweglichen Bett von einem ersten Bereich eines medizinischen Zentrums zu einem zweiten Bereich des medizinischen Zentrums zu bewegen, wo sich der medizinische Bildgebungsscanner befindet;
wobei der medizinische Bildgebungsscanner dazu vorgesehen ist, zumindest ein medizinisches Bild des Patienten zu erfassen;
wobei der zumindest eine Sensor dazu konfiguriert ist, Sensorinformationen über den Patienten auf dem beweglichen Bett zu erfassen, und wobei der zumindest eine Sensor dazu konfiguriert ist, die Sensorinformationen über den Patienten auf dem beweglichen Bett zu erfassen, bevor der Patient von dem ersten Bereich des medizinischen Zentrums in den zweiten Bereich des medizinischen Zentrums bewegt wird und/oder während der Patient von dem ersten Bereich des medizinischen Zentrums in den zweiten Bereich des medizinischen Zentrums bewegt wird;
wobei der zumindest eine Sensor dazu konfiguriert ist, die Sensorinformationen an die Prozessoreinheit bereitzustellen;
wobei der medizinische Bildgebungsscanner dazu konfiguriert ist, Scannerstatusinformationen für den medizinischen Bildgebungsscanner an die Prozessoreinheit bereitzustellen;
**dadurch gekennzeichnet, dass**
die Prozessoreinheit dazu konfiguriert ist, Steuerinformationen für das bewegliche Bett zu bestimmen, wobei die Bestimmung die Nutzung der Sensorinformationen und der Scannerstatusinformationen umfasst; und
dass die Prozessoreinheit dazu konfiguriert ist, die Steuerinformationen zu nutzen, um eine Mitteilung bereitzustellen, dass eine Bedienungsperson zu dem beweglichen Bett kommen muss, und/oder die Prozessoreinheit dazu konfiguriert ist, die Steuerinformationen zu nutzen, um eine Mitteilung bereitzustellen, dass eine Bedienungsperson das bewegliche Bett bewegen muss, und/oder die Prozessoreinheit dazu konfiguriert ist, die Steuerinformationen zu nutzen, um eine Mitteilung bereitzustellen, dass eine Bedienungsperson die Bewegung des beweglichen Betts stoppen muss.

2. System nach Anspruch 1, wobei die Sensorinformationen über den Patienten auf dem beweglichen Bett Lebenszeichen des Patienten auf dem beweglichen Bett umfassen.

3. System nach Anspruch 2, wobei die Lebenszeichen eines oder mehrere der folgenden umfassen: EKG-Daten; Anästhesiedaten; Herzschlagdaten; Blutdruckdaten; Hautleitfähigkeitsdaten; Atemfrequenzdaten; Bewegungsdaten von Körperteilen, die aus HF- und/oder optischer Sensorik bestimmt wurden.

4. System nach einem der Ansprüche 1-3, wobei der medizinische Bildgebungsscanner eine MRT-Bildeinheit, eine Röntgenbildeinheit, eine PET-Bildeinheit, eine CT-Strahlentherapieeinheit oder ein MR-LINAC-Scanner ist.

5. System nach Anspruch 4, wobei der medizinische Bildgebungsscanner eine MRT-Bildeinheit ist, und wobei der zumindest eine Sensor zumindest eine drahtlose MRT-kompatible Spule umfasst, und wobei die Sensorinformationen über den Patienten auf dem beweglichen Bett Statusinformationen über die zumindest eine drahtlose MRT-kompatible Spule umfassen.

6. System nach Anspruch 5, wobei die Statusinformationen über die zumindest eine drahtlose MRT-kompatible Spule eines oder mehrere der folgenden umfassen: Batteriestatus der drahtlosen MRT-kompatiblen Spule oder Spulen; Typ der Spule oder Spulen; den Spulenhersteller oder die Spulenhersteller; HF-Leistungsgrenze der Spule oder Spulen; Betriebstemperatur der Spule oder Spulen; Servicedaten der Spule oder Spulen; Funktionalitätsdaten der Spule oder Spulen.

7. System nach einem der Ansprüche 5-6, wobei die Steuerinformationen für das bewegliche Bett Spulenkonfigurationsinformationen umfassen, um die drahtlose MRT-kompatible Spule im Hinblick auf die Erfassung des zumindest einen medizinischen Bildes des Patienten zu konfigurieren.

8. System nach einem der Ansprüche 1-7, wobei die Prozessoreinheit dazu konfiguriert ist, Konfigurationsinformationen für den medizinischen Bildgebungsscanner zu bestimmen, wobei die Bestimmung Nutzung der Sensorinformationen und der Scannerstatusinformationen umfasst.

9. System nach einem der Ansprüche 1-8, wobei die Scannerstatusinformationen für den medizinischen Bildgebungsscanner einen Hinweis darauf, ob der Scanner in Gebrauch ist und/oder einen Hinweis darauf umfassen, wann der Scanner zur Nutzung bereitsteht.

10. System nach einem der Ansprüche 1-9, wobei das System ein oder mehrere weitere bewegliche Betten umfasst, und wobei die Scannerstatusinformationen Sensorinformationen über einen oder mehrere andere Patienten auf dem einen oder den mehreren weiteren beweglichen Betten umfassen, und wobei der medizinische Bildgebungsscanner dazu vorgesehen ist, für jeden des einen oder der mehreren anderen Patienten zumindest ein medizinisches Bild zu erfassen.

11. System nach einem der Ansprüche 1-10, wobei die Steuerinformationen Anweisungen zum Steuern einer oder mehrerer Bremsen für ein oder mehrere Räder des beweglichen Betts umfassen.

12. System nach einem der Ansprüche 1-11, wobei die Steuerinformationen Anweisungen zum Steuern der Bereitstellung von Bewegungsinformationen an eine Bedienungsperson umfassen, wobei die Bewegungsinformationen Informationen umfassen, welche auf zumindest einer visuellen Anzeigeeinheit angezeigt werden; und/oder wobei die Bewegungsinformationen eine Informationsausgabe auf zumindest einer akustischen Ausgabeeinheit umfassen.

13. System nach Anspruch 12, wobei die zumindest eine visuelle Anzeigeeinheit eine an dem beweglichen Bett montierte visuelle Anzeigeeinheit umfasst; und/oder wobei die zumindest eine visuelle Anzeigeeinheit eine von dem beweglichen Bett getrennte visuelle Anzeigeeinheit umfasst; und/oder wobei die zumindest eine akustische Ausgabeeinheit eine an dem beweglichen Bett montierte akustische Ausgabeeinheit umfasst; und/oder wobei die zumindest eine akustische Ausgabeeinheit eine von dem beweglichen Bett getrennte akustische Ausgabeeinheit umfasst.

14. Medizinisches Bildgebungsverfahren (100) mit einem medizinischen Bildgebungssystem (10), wobei das Verfahren
umfasst:
a) Bewegen (110) eines Patienten auf einem beweglichen Bett von einem ersten Bereich eines medizinischen Zentrums zu einem zweiten Bereich des medizinischen Zentrums, wo sich ein medizinischer Bildgebungsscanner befindet, und wobei der medizinische Bildgebungsscanner dazu vorgesehen ist, zumindest ein medizinisches Bild des Patienten zu erfassen;
b) vor dem Bewegen des Patienten und/oder während der Bewegung des Patienten, Erfassen (120) von Sensorinformationen über den Patienten auf dem beweglichen Bett durch zumindest einen Sensor;
c) Bereitstellen (130) der Sensorinformationen an eine Prozessoreinheit;
d) vor dem Bewegen des Patienten und/oder während der Bewegung des Patienten, Bereitstellen (140) von Scannerstatusinformationen für den medizinischen Bildgebungsscanner an die Prozessoreinheit; und
e) vor dem Bewegen des Patienten und/oder während der Bewegung des Patienten, Bestimmen (150) von Steuerinformationen für das bewegliche Bett durch die Prozessoreinheit, wobei das Bestimmen das Nutzen der Sensorinformationen und der Scannerstatusinformationen umfasst, und Nutzen der Steuerinformationen durch die Verarbeitungseinheit, um eine Mitteilung bereitzustellen, dass eine Bedienungsperson zu dem beweglichen Bett kommen muss, und/oder Nutzen der Steuerinformationen durch die Verarbeitungseinheit, um eine Mitteilung bereitzustellen, dass eine Bedienungsperson das bewegliche Bett bewegen muss, und/oder Nutzen der Steuerinformationen durch die Verarbeitungseinheit, um eine Mitteilung bereitzustellen, dass eine Bedienungsperson die Bewegung des beweglichen Betts stoppen muss.

15. Computerprogrammelement zum Steuern eines Systems nach einem der Ansprüche 1 bis 13, das, wenn es von einem Prozessor ausgeführt wird, dazu konfiguriert ist, das Verfahren nach Anspruch 14 durchzuführen.

## Revendications

1. Système d'imagerie médicale (10), comprenant :
- un lit mobile (20) ;
- un scanner d'imagerie médicale (30) ;
- au moins un capteur (40) ; et
- une unité de traitement (50) ;
dans lequel le lit mobile est configuré pour déplacer un patient sur le lit mobile d'une première zone d'un centre médical vers une seconde zone du centre médical dans laquelle le scanner d'imagerie médicale se trouve ;
dans lequel le scanner d'imagerie médicale est destiné à acquérir au moins une image médicale du patient ;
dans lequel le au moins un capteur est configuré pour acquérir des informations de capteur se rapportant au patient sur le lit mobile et dans lequel le au moins un capteur est configuré pour acquérir les informations de capteur se rapportant au patient sur le lit mobile avant un déplacement du patient de la première zone du centre médical à la seconde zone du centre médical et/ou pendant un déplacement du patient de la première zone du centre médical à la seconde zone du centre médical ;
dans lequel le au moins un capteur est configuré pour fournir les informations de capteur à l'unité de traitement ;
dans lequel le scanner d'imagerie médicale est configuré pour fournir des informations d'état de scanner pour le scanner d'imagerie médicale à l'unité de traitement ;
**caractérisé en ce que**
l'unité de traitement est configurée pour déterminer des informations de commande pour le lit mobile, la détermination comprenant une utilisation des informations de capteur et des informations d'état de scanner ; et
**en ce que** l'unité de traitement est configurée pour utiliser les informations de commande pour fournir une communication indiquant qu'un opérateur doit se rendre au lit mobile et/ou l'unité de traitement est configurée pour utiliser les informations de commande pour fournir une communication indiquant qu'un opérateur doit déplacer le lit mobile et/ou l'unité de traitement est configurée pour utiliser les informations de commande pour fournir une communication indiquant qu'un opérateur doit arrêter de déplacer le lit mobile.

2. Système selon la revendication 1, dans lequel les informations de capteur se rapportant au patient sur le lit mobile comprennent des données de signes vitaux pour le patient sur le lit mobile.

3. Système selon la revendication 2, dans lequel les données de signes vitaux comprennent un ou plusieurs éléments parmi : des données ECG ; des données d'anesthésie ; des données de rythme cardiaque ; des données de pression artérielle ; des données de conductance cutanée ; des données de fréquence respiratoire ; des données de déplacement de parties de corps déterminées par détection RF et/ou optique.

4. Système selon l'une quelconque des revendications 1-3, dans lequel le scanner d'imagerie médicale est une unité d'image IRM, une unité d'image à rayons X, une unité d'image PET, une unité de radiothérapie CT ou un scanner MR-LINAC.

5. Système selon la revendication 4, dans lequel le scanner d'imagerie médicale est une unité d'image IRM, et dans lequel le au moins un capteur comprend au moins une bobine compatible IRM sans fil,et dans lequel les informations de capteur se rapportant au patient sur le lit mobile comprennent des informations d'état se rapportant à la au moins une bobine compatible IRM sans fil.

6. Système selon la revendication 5, dans lequel les informations d'état se rapportant à la au moins une bobine compatible IRM sans fil comprennent un ou plusieurs éléments parmi : l'état de batterie de la ou des bobines compatibles IRM sans fil ; le type de la ou des bobines ; le ou les fournisseurs de bobine ; la limite de puissance RF de la ou des bobines ; la température de fonctionnement de la ou des bobines ; des données de service de la ou des bobines ; des données de fonctionnalité de la ou des bobines.

7. Système selon l'une quelconque des revendications 5-6, dans lequel les informations de commande pour le lit mobile comprennent des informations de configuration de bobine pour configurer la bobine compatible IRM sans fil par rapport à l'acquisition de la au moins une image médicale du patient.

8. Système selon l'une quelconque des revendications 1-7, dans lequel l'unité de traitement est configurée pour déterminer des informations de configuration pour le scanner d'imagerie médicale, la détermination comprenant une utilisation des informations de capteur et des informations d'état de scanner.

9. Système selon l'une quelconque des revendications 1-8, dans lequel les informations d'état de scanner pour le scanner d'imagerie médicale comprennent une indication indiquant si le scanner est, ou non, en cours d'utilisation et/ou une indication du moment pendant lequel le scanner sera disponible à l'emploi.

10. Système selon l'une quelconque des revendications 1-9, dans lequel le système comprend un ou plusieurs autres lits mobiles et dans lequel les informations d'état de scanner comprennent des informations de capteur se rapportant à un ou plusieurs autres patients sur les un ou plusieurs autres lits mobiles et dans lequel le scanner d'imagerie médicale est destiné à acquérir au moins une image médicale pour chacun des un ou plusieurs autres patients.

11. Système selon l'une quelconque des revendications 1-10, dans lequel les informations de commande comprennent des instructions pour commander un ou plusieurs freins pour une ou plusieurs roues du lit mobile.

12. Système selon l'une quelconque des revendications 1-11, dans lequel les informations de commande comprennent des instructions pour commander la fourniture d'informations de déplacement à un opérateur, dans lequel les informations de déplacement comprennent des informations affichées sur au moins une unité d'affichage visuel ; et/ou dans lequel les informations de déplacement comprennent des informations émises sur au moins une unité de sortie acoustique.

13. Système selon la revendication 12, dans lequel la au moins une unité d'affichage visuel comprend une unité d'affichage visuel montée sur le lit mobile ; et/ou dans lequel la au moins une unité d'affichage visuel comprend une unité d'affichage visuel séparée du lit mobile ; et/ou dans lequel la au moins une unité de sortie acoustique comprend une unité de sortie acoustique montée sur le lit mobile ; et/ou dans lequel la au moins une unité de sortie acoustique comprend une unité de sortie acoustique séparée du lit mobile.

14. Procédé d'imagerie médicale (100) avec un système d'imagerie médicale (10), le procédé comprenant :
a) le déplacement (110) d'un patient sur un lit mobile d'une première zone d'un centre médical à une seconde zone du centre médical dans laquelle un scanner d'imagerie médicale se trouve, et dans lequel le scanner d'imagerie médicale est destiné à acquérir au moins une image médicale du patient ;
b) avant le déplacement du patient et/ou pendant le déplacement du patient, l'acquisition (120), par au moins un capteur, d'informations de capteur se rapportant au patient sur le lit mobile ;
c) la fourniture (130) des informations de capteur à une unité de traitement ;
d) avant le déplacement du patient et/ou pendant le déplacement du patient, la fourniture (140) d'informations d'état de scanner pour le scanner d'imagerie médicale à l'unité de traitement ; et
e) avant le déplacement du patient et/ou pendant le déplacement du patient, la détermination (150), par l'unité de traitement, d'informations de commande pour le lit mobile, la détermination comprenant l'utilisation des informations de capteur et des informations d'état de scanner, et l'utilisation, par l'unité de traitement, des informations de commande pour fournir une communication indiquant qu'un opérateur doit se rendre au lit mobile et/ou l'utilisation, par l'unité de traitement, des informations de commande pour fournir une communication indiquant qu'un opérateur doit déplacer le lit mobile et/ou l'utilisation, par l'unité de traitement, des informations de commande pour fournir une communication indiquant qu'un opérateur doit arrêter de déplacer le lit mobile.

15. Élément de programme informatique pour commander un système selon l'une quelconque des revendications 1 à 13, qui, lorsqu'il est exécuté par un processeur, est configuré pour mettre en œuvre le procédé selon la revendication 14.
